# EUROPEAN PATENT SPECIFICATION

(11) **EP 1 618 855 B1**
(45) Date of publication and mention of the grant of the patent: **09.12.2015**
(21) Application number: 05254564.7
(22) Date of filing: 21.07.2005
(51) Int. Cl.: A61F 2/01

(54) **Device for filtering blood in a vessel with helical elements**
Vorrichtung zum Filtern von Blut mit schraubenförmigen Elementen
Dispositif à éléments hélicoïdaux de filtrage du sang

(30) Priority: 22.07.2004 US 896571
(43) Date of publication of application: 25.01.2006
(73) Proprietor: Cordis Corporation, Fremont, CA 94555 (US)
(72) Inventor: Niermann, Volker, Bound Brook NJ 08805 (US)
(74) Representative: Small, Gary James

(56) References cited:
- FR-A- 2 512 678
- US-A- 4 643 184
- US-A- 4 760 849
- US-A- 5 129 910
- US-A- 5 160 342
- US-A1- 2003 014 127

## Description

In the human cardiovascular and circulatory system, the consistency of blood remains liquid enough for the blood cells and other molecules to travel smoothly through the arteries and veins. Sometimes, however, clots will form in a process called coagulation. When clots or other blood-borne clumps of tissue migrate through the circulatory system, they are called emboli; a single migrating clot is called an embolus or an embolism.

A pulmonary embolism is a clot that travels through the venous system and eventually lodges in the pulmonary artery, which carries blood from the heart to the lungs. This can obstruct the blood supply to the lungs, which is potentially fatal and should be treated as an emergency.

Many pulmonary emboli result from a condition called deep vein thrombosis (DVT). DVT is the formation of a blood clot in the veins embedded deep in the muscles, usually in the lower leg and sometimes in the pelvis or groin.

Vena cava filters, tiny nets, help prevent emboli from traveling through the heart and into the lungs. Most commonly, vena cava filters are inserted into the inferior vena cava, a large vein that carries blood from the lower extremities.

Vena cava filters are normally metallic, umbrella-shaped devices that catch blood clots to prevent them from traveling to the lungs and causing a pulmonary embolism. Vena cava filters usually are used when drug therapy, such as treatment with blood-thinners, has failed or is considered inadequate, or when drug therapy would cause other dangerous medical conditions.

The procedure is safe and effectively reduces the risk of pulmonary embolism in most people when performed by a practitioner who is skilled in filter insertion and when complemented by drug therapies.

People most likely to receive a vena cava filter are those at risk for pulmonary embolism and those for whom drug or other therapy is considered inadequate. Vena cava filters are also inserted to protect trauma patients from pulmonary embolism associated with their injuries.

The procedure for placing a vena cava filter in a patient usually requires that the physician administer a local anesthetic at the insertion site, either the arm, neck, or groin, and makes an incision. Patients may also receive a muscle relaxant for additional comfort. Alternatively, the procedure may be performed while the patient is under general anesthesia.

The physician then inserts the collapsed filter into the incision via a catheter (a long, thin, flexible tube) and advances the filter to the vena cava. The physician then deploys the filter in the vein at the target location, removes the insertion device, and closes the incision. The procedure generally takes from 10 to 40 minutes. Antibiotics are prescribed as necessary to minimize the risk of infection.

Patients are likely to remain in the hospital until the supervising physician confirms that the filter is properly fixed in the vena cava and that there are no complications from the procedure. The presence of a vena cava filter does not affect daily routines or the use of other medications. Some patients may remain on anticoagulant drug therapy to reduce the risk of post-insertion clot formation, or risk enlarging a pre-existing clot.

However, there are known complications that may arise in any vena cava filter placement even though known vena cava filters are about 98 percent successful in preventing symptomatic pulmonary embolism. These known filter devices and their placement procedures can be associated with surgical and anesthesia complications to include: bleeding at the insertion site; anesthesia-associated complications such as an allergic reaction or breathing problems; stroke; pulmonary embolism; and clots. And, as is well known in the field, these complications are not only serious to the patient's health, but they can also be fatal.

Thrombosis of the inferior vena cava (IVC) filter after filter placement is frequently reported and may occur with all types of filter presently used in the field. The occurrence of thrombosis can be delayed from hours to several months after the filter placement, but seems more frequent during the first 3 months. Continued anticoagulation therapy has not been shown to prevent IVC thrombosis.

Studies have also shown adverse flow dynamics, such as increased pressure gradients, in the filters with high clot-trapping capacity. Accordingly a device that has a high clot capture efficiency while minimizing the potential for increased pressure gradient is desirable.

Accordingly, what is needed is a device and method that can further reduce these serious and fatal complications in a more reliable and predictable manner. To date, there have been no known filter devices that are designed in such a manner that can eliminate these complications on a consistent basis, particularly providing for the elimination of complications that may be attributed to pulmonary embolism and blood clots.

US 4,760,849 discloses a device for capturing an embolus within a vessel of a patient's body, the device being of the type defined in the preamble of the accompanying claim 1.

The present invention is a novel fitter device for filtering blood in a vessel that is more highly effective in capturing clots and preventing pulmonary embolism over the known prior art devices and techniques. Although the invention is described below in connection with a filtering procedure, no claim is made herein to a method of surgery.

### SUMMARY OF THE INVENTION

The present invention is a novel filter device for filtering fluid or blood in a vessel or organ that is more highly effective in capturing clots, emboli, particulate matter and particles and preventing pulmonary embolism over the known prior art devices and techniques The device will also avoid plugging up and restricting blood flow.

The present invention is directed to various embodiments of devices for trapping or capturing emboli in a vessel of patient's body or organ, as defined by the claims.

In all embodiments of the present invention, pore sizes can vary. All pore sizes have a size ≥ 120 µm. Moreover, in all embodiments of the present invention, the pore sizes of the device can vary from one end of the device to an opposite end of the device. For example, the pore size can vary from a larger size pore at one end of the device to a smaller size pore at an opposite end of the device wherein the pore size decreases throughout the entire length of the device, i.e. pore size decreases from the one end to the opposite end of the device such as found with depth type filter devices. The at least one helix having a plurality of turns helically arranged around a longitudinal axis can vary in pitch. This pitch may decrease to zero, to the point where the helix ends by making a full revolution and contacts itself. Additionally, in all embodiments of the present invention, the pore size can be a uniform size throughout the device, i.e. from one end of the device to the opposite end of the device.

### BRIEF DESCRIPTION OF THE DRAWINGS

The novel features of the invention are set forth with particularity in the appended claims. The invention itself, however, both as to organization and methods of operation, together with further objects and advantages thereof, may be understood by reference to the following description, taken in conjunction with the accompanying drawings in which:
Fig. 1A is a schematic illustration of a vessel in cross-section having a helical filter device for capturing emboli in accordance with the present invention;
Fig. 1B is an enlarged illustration of a portion of the vessel and filter device of Fig. 1 capturing emboli therein in accordance with the present invention;
Fig. 2A is a schematic illustration of another embodiment of the filter device of Figs. 1A and 1B in accordance with the present invention;
Fig. 2B is a schematic illustration of the filter device of Fig. 2A having a plurality of anchoring mechanisms for securing the device to the inner wall of a vessel or organ in accordance with the present invention;
Fig. 3A is a schematic illustration of another embodiment of the filter device of Figs. 1A and 1B having varying pore sizes extending from one end of the device to an opposite end thereof and also including an optional spine in accordance with the present invention;
Fig. 3B is a schematic illustration of the filter device of Fig. 3A having a plurality of anchoring mechanisms for securing the device to the inner wall of a vessel or organ in accordance with the present invention;
Fig. 4A is a schematic illustration of another embodiment of the filter device of Figs. 1A and 1B having a double helix design in accordance with the present invention;
Fig. 4B is a schematic illustration of the filter device of Fig. 4A having a plurality of anchoring mechanisms for securing the device to the inner wall of a vessel or organ in accordance with the present invention;
Figs. 5A, 5B and 5C are schematic illustrations of a manufacturing method and method for expanding the filter device of Figs 1A and 1B in accordance with the present invention; and
Fig. 6 is a schematic illustration of the filter device and device for delivering the filter device in accordance with the present invention.

### DESCRIPTION OF THE PREFERRED EMBODIMENTS

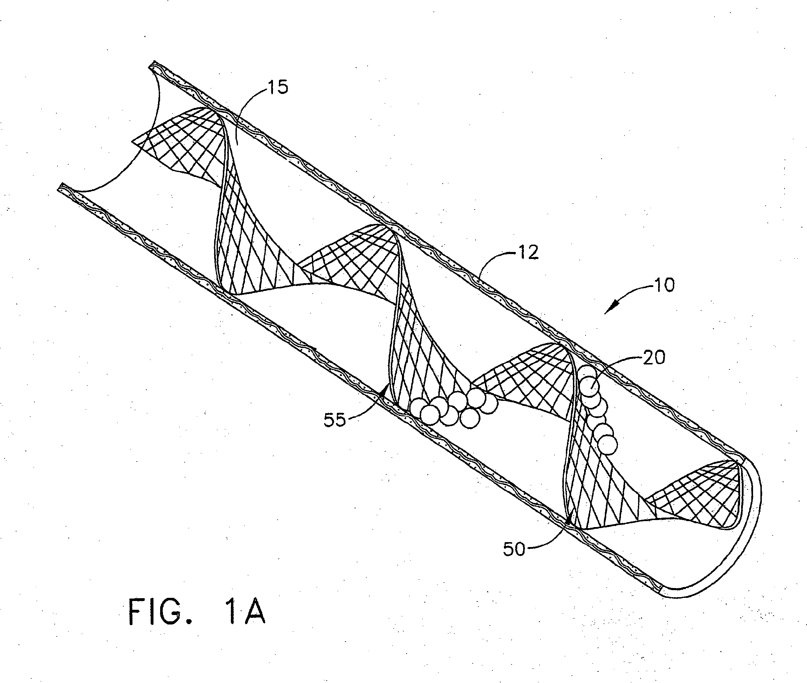

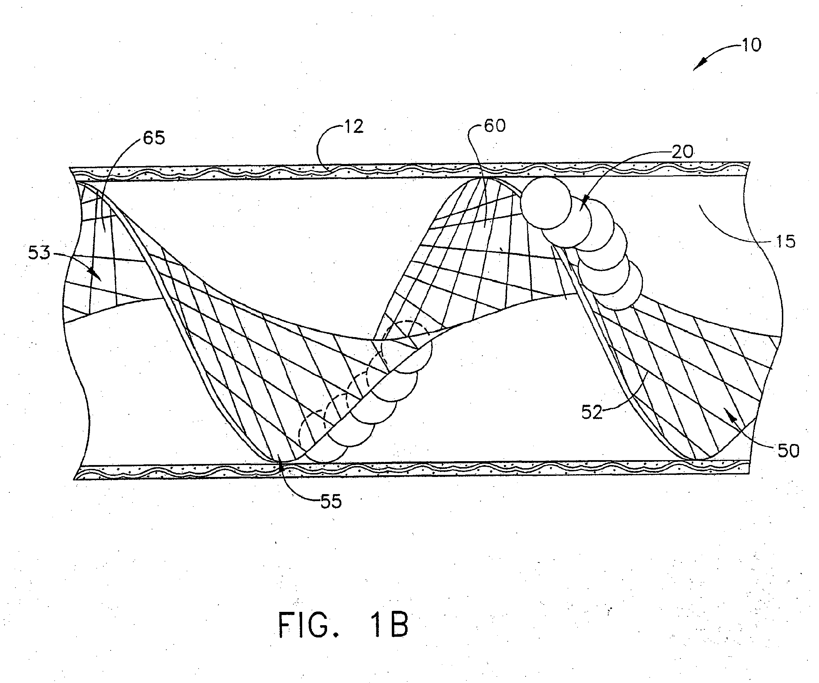

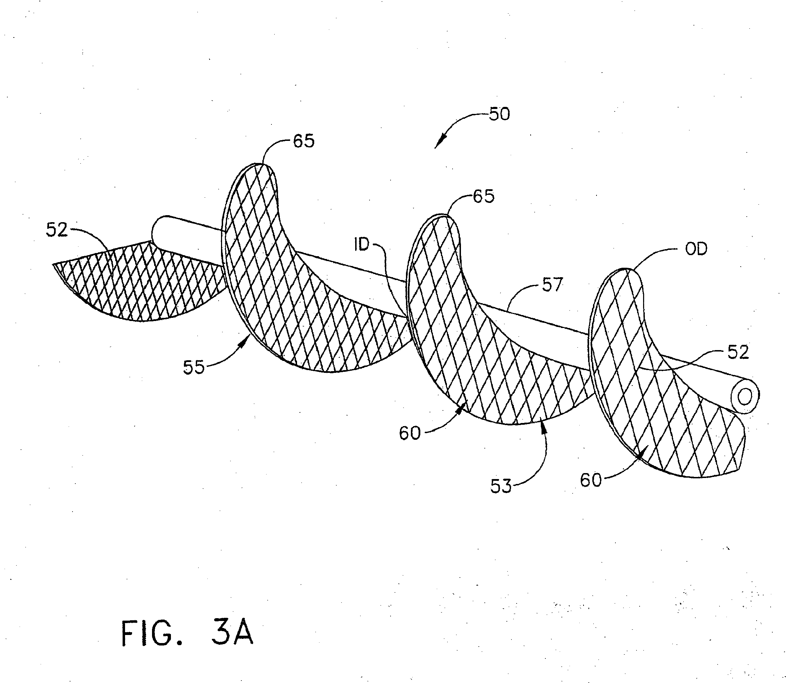

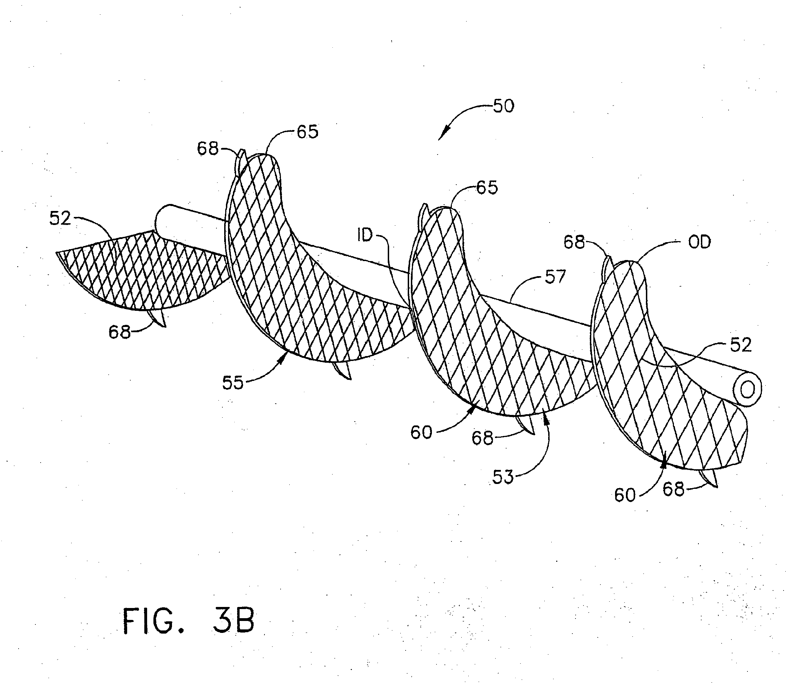

The present invention is a filter device, generally designated 50, having ahelical design that is either surface type filter (Figs 1A, 1 B, 2A, 2B, 4A and 4B) or depth type filter (Figs 3A and 3B) that may be employed in any generally cylindrical pathway such as a vessel 10 (Figs. 1A and 1B) such as a vein or artery, for example the vena cava, or a duct or an organ of the human body. The filter device 50 and method for using the device 50 is particularly useful for filtering a vena cava and more particularly useful for treatment of vascular disease such as DVT although the device 50 and method of using same is not in any way limited to this particular anatomy or disease state.

The filter device 50 has a helix 55 (as either a single helix or double helix as better described later on below) that is particularly useful for trapping and capturing clots, emboli, particulate matter, particles and thrombus that are migrating or circulating throughout the circulatory system of the patient or are in danger of breaking apart from attached tissue or structure within the body and migrating or circulating throughout the circulatory system of the patient. As defined herein, the term "clot", "clots", "embolus", "embolism", "emboli", "particulate", "particulate matter", "matter", "particles", "filtrate", "thrombus", and "thrombi" have the same meaning for purposes of this disclosure and are used interchangeably throughout and are generally designated as reference numeral 20.

The helix 55 of filter device 50 is made of a mesh material 52 having a plurality of pores 53 throughout the mesh 52. For example, the mesh 52 consists of a plurality of interlocking strands or fibers or an array of pores 53 made and arranged in the material 52 itself such as through cutting, etching, stamping or the like. Details for the pores 53 are addressed below.

The material 52 is any form of material. In one embodiment, the material 52 is a self-expanding material such as shape-memory material which can be a metal alloy such as nickel titanium alloy (nitinol). In another embodiment, the material 52 is a stainless steel alloy. Alternatively, the mesh material 52 is a polymer material. The polymer can be biodegradable and/or bioabsorbable. As used herein, the term "biodegradable" is defined as the breaking down or the susceptibility of a material or component to break down or be broken into products, byproducts, components or subcomponents over time such as days, weeks, months or years. As used herein, the term "bioabsorbable" is defined as the biologic elimination of any of the products of degradation by metabolism and/or excretion.

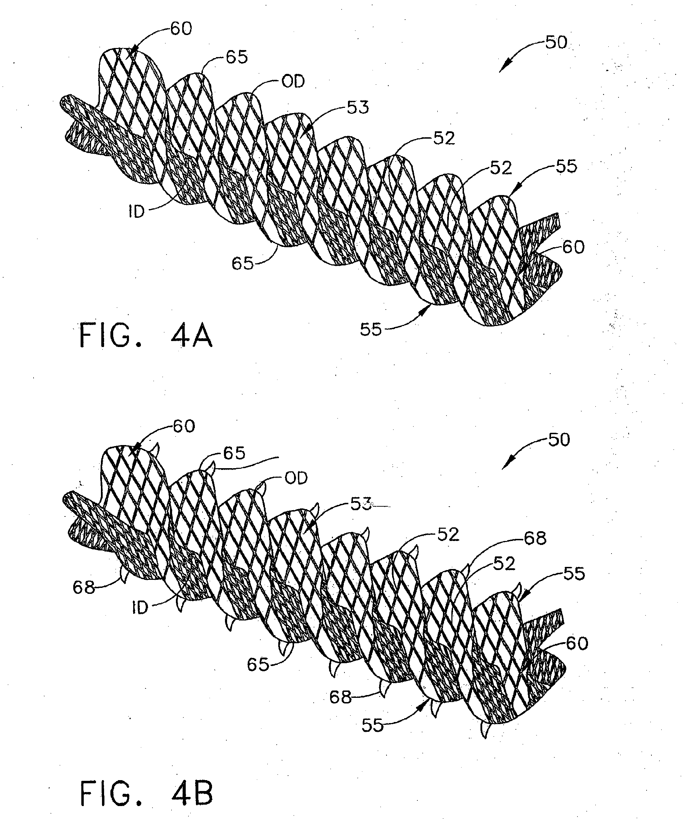

The expanded shape of the filter 50 comprises at least one helix 55, for example a single helix (Figs 1A, 1B, 2A, 2B, 3A and 3B) or a double helix (Figs. 4A and 4B). The single helix 55 and double helix 55 respectively in some embodiments of the invention comprise a plurality of pleats or panels 60 helically arranged around a longitudinal axis of the device 50. The panels 60 are helically arranged around the longitudinal axis in a plurality of helical turns 65. The helical turns 65 define an inner diameter (ID) and an outer diameter (OD) respectively. Alternatively, the helix 55 of the device 50 is constructed of a single piece of mesh material 52 or discrete sections of mesh 52 fused or connected to each other forming the single helix or double helix (Figs 4A and 4B) of the filter device 50. The helical turns 65 of filter device have uniform pitch, or alternatively have a variable pitch depending on the channeling effect desired by the end user.

It is preferable that the mesh 52 of each turn 65 is sloped, slanted, inclined or curved away from ID of helix 55 to OD of helix 55 such as depicted in the Figs., or alternatively, the helix 55 may have no incline or be inclined toward the longitudinal axis. Since the mesh 52 is slanted or curved outwardly from 1D to OD for each turn 65 of helix 55, fluid medium is forced and channeled toward the outer circumferential periphery of the helix 55. The panels 60 design for the helix 55 in the embodiments depicted in Figs. 1A and 1B facilitate this outward inclined feature and outward fluid channeling effect.

The helix 55 has a plurality of turns 65 helically arranged around a longitudinal axis that can vary in pitch. This pitch may decrease to zero, to the point where the helix 55 ends or terminates by making a full revolution and contacts itself.

In some embodiments according to the present invention, the helix 55 includes a spine 57 as best illustrated in Figs 3A and 3B. The spine 57 serves as a central longitudinal shaft or axis for the helical turns 65 of the helix 55. The spine 57 is optional for the helix 55 since the helix 55 can be constructed without this feature.

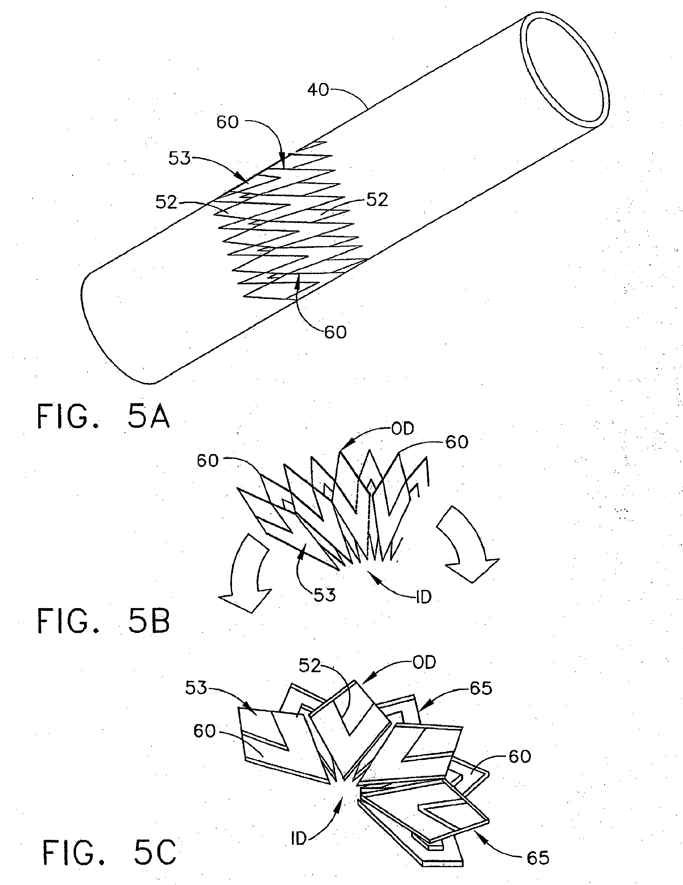

The filter device 50 is expandable from a compressed, closed, pre-deployed or collapsed state to an open, deployed or expanded state such as partially depicted in Figs 5B and 5C. For those embodiments having a plurality of panels 60 such as depicted in Figs. 1A and 1 B, the panels 60 of mesh 52 circumferentially expand upon deployment of the device 50 as best shown by direction arrows in Fig. 5B. The filter device 50 is introduced into a lumen 15 of the vessel 10 in the compressed, closed, pre-deployed or collapsed state and the device 50 is deployed in the lumen 15 of the vessel 10 by movable expansion of the helix 55 to the open, deployed or expanded state. When moved to the open, deployed or expanded state, the ID of the helix 55 roughly aligns along the longitudinal axis of the vessel 10 and the OD of the helix 55 is adjacent inner wall 12 of the vessel 10.

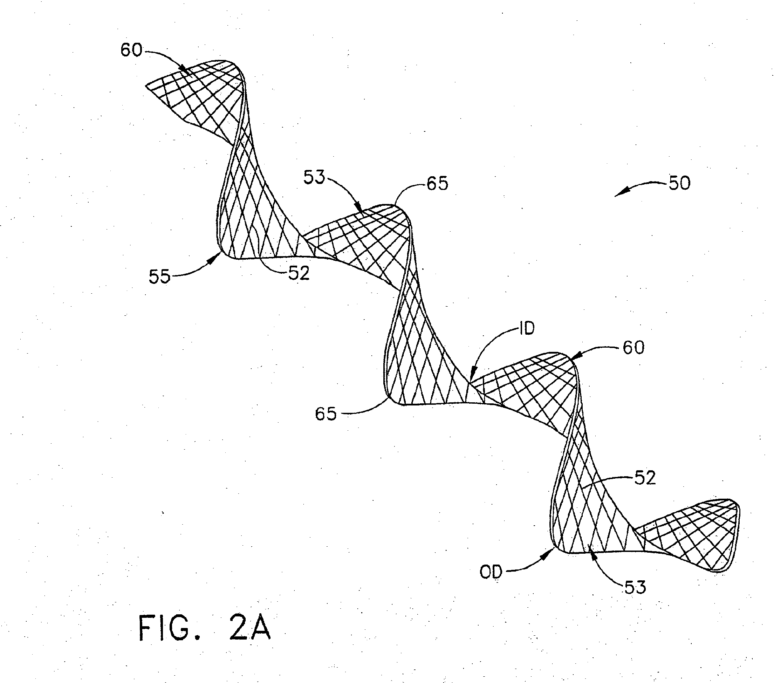

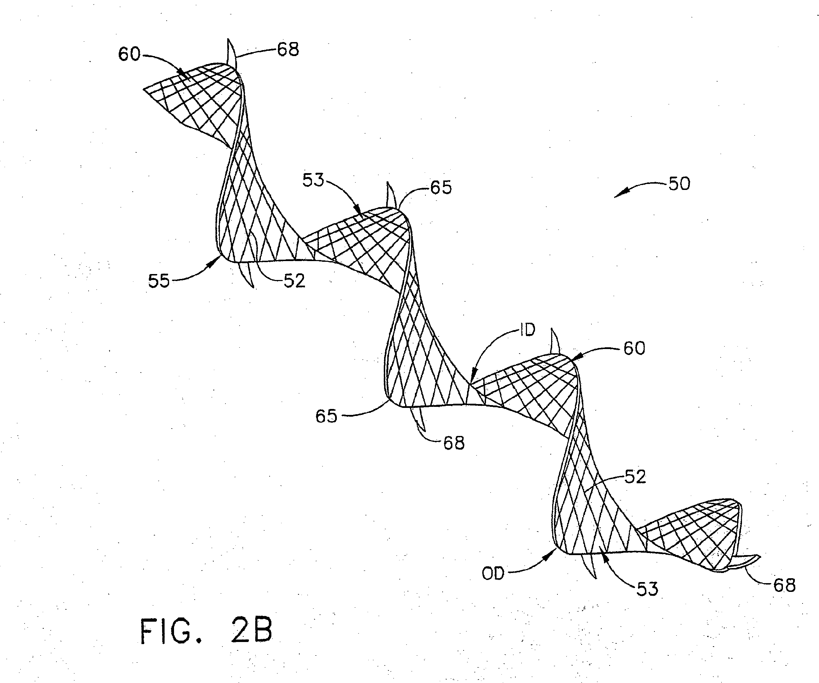

Additionally, when moved to the open, deployed or expanded state, the helix 55 embeds itself in the wall 12 of the vessel 10 such as shown in Figs 1A and 1B. As best illustrated in Figs. 2B, 3B, and 4B, anchoring mechanisms 68, such as a plurality of barbs 68, are used to secure the helix 55 in tissue such as the wall 12 of vessel 10.

The size for each pore 53 is ≥ 120mm. Additionally, in all embodiments of the present invention, the pore size can be a uniform size throughout the entire length of the device 50, i.e. from one end of the device 50 to the opposite end of the device 50.

The filter device 50 according to the present invention (all embodiments) provides the ability to expose a greater surface area of the filter device 50 due to the unique helix 55 feature. Based on its helical design, the filter device 50 permits a smaller pore structure 53 (over the known filters and filtering methods) because the possibility of stopping venous flow is eliminated. Accordingly, smaller sized clots 20, for instance clots 20 having a size ≥ 120 µm, can be targeted and captured, thereby reducing risk to the patient, i.e. the risk of these smaller size clots 20 causing harm.

Moreover, in all embodiments of the present invention, the pore sizes of the filter device 50 can vary from one end of the device 50 to an opposite end of the device 50. For example, as best illustrated in Figs 3A and 3B, the pore size can vary from a larger size pore at one end of the device (for example a 5mm pore size) to a smaller size pore 53 at an opposite end of the device 50 (for example a 120 µm pore size) such that the pore size decreases throughout the entire length of the device 50, i.e. pore size decreases from the one end to the opposite end of the device 50 thereby increasing the useful life of the device 50 such as found with depth type filter devices. The larger clots 20 are captured at the beginning of the helix 55 of filter device 50 reserving the smaller pore structure portion at opposite or far end of the helix 55 of filter device 50 to remove the smaller clots 20.

The structure of the helix 55 is an expanded mesh 52 that creates the surface filter effect. Any particulate or clot 20 that approaches the filter device 50 according to the present invention encounters what appears to be a solid cylindrical impediment in the lumen 15 of vessel 10 (since OD of helix 55 circumferentially is expanded to and circumferentially conforms to inner wall 12 of vessel 10 as best shown in Figs 1A and 1B). However the helical twist of helix 55 allows lower viscosity fluid medium (such as blood) to flow through pores 53 and around the mesh 52. Any particulate or clot 20 present in this fluid flow will impinge the mesh 52 of the helix 55 and either be trapped there, or be forced out toward the outer periphery of the helix 55 by a helical centrifugal flow effect. The helical structure of the filter device 50 according the present invention also induces outward force by the outward curvature or inclination of the mesh 52 where the particulate or clot 20 will be trapped. The fluid (blood) is free to move around and passed the clot 20, even if the filter structure is fully covered by particulate or clots 20.

There are several advantages to the helical filter design of the filter device 50 according to the present invention, for example, the ability of the helix 55 of filter device 50 to filter large amounts of filtrate (clots 20) and completely avoid clogging or plugging the lumen 15 of vessel 10, i.e. vena cava 10 in this example. This is especially important since prior art filters increase the resistance in the lumen 15 of vessel 10 as they are eventually clogged or plugged by particulate matter (clots 20), eventually restricting the flow within vessel 10 thereby cutting off or occluding fluid flow altogether.

The helical filter design of filter device 50 of the present invention captures the filtrate 20 by inertial impaction, or diverts it to the outside edges or periphery of the helix 55 thereby trapping it, while allowing the fluid medium (liquid or gas) to pass around the new obstruction created by the captured filtrate 20.

Other advantages of the filter device 50 of the present invention include the ability to generate a filter having different pore sizes from beginning to end as depicted in Figs. 3A and 3B, mimicking a depth type filter, thereby increasing the filter life. This variable pore size (along the length of the device 50) feature ensures that larger clots 20 will be captured at the beginning of the filter where the size of pores 53 are larger, reserving the smaller pore structure portion of the filter to remove the smaller clots 20.

Other advantages for the filter device 50 of the present invention relate to its delivery, deliverability and manufacturability. For example, as depicted in Fig. 5A, for those embodiments of the present invention made of shape memory material, such as nickel titanium as one example, the shape memory alloy is used as the structure of the filter 50 itself and will also serve as the delivery mechanism for the filter 50 as better described below.

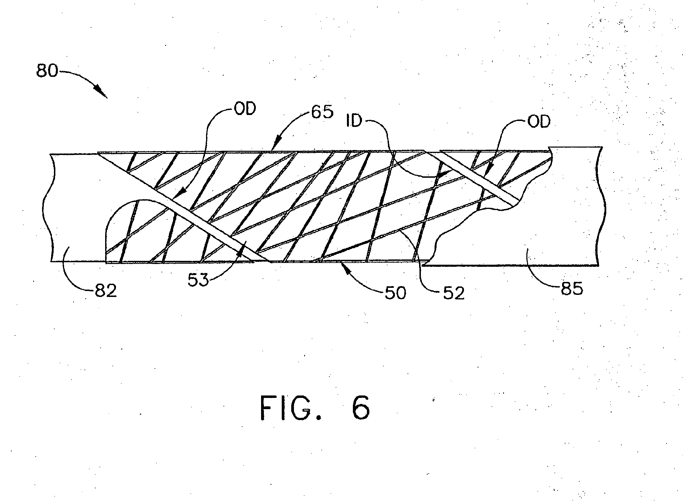

As shown in Fig. 5A, the filter device 50 can be laser cut in the general shape of a ribbon out of a tube 40 of shape memory material (nickel titanium in this example). The final cut shape taken from shape memory tube 40 is generally akin to a ribbon as best shown in Fig. 6. The cut device 50 (ribbon-like at this point) is loaded onto a shaft 82 of a catheter 80 which is akin to taking a ribbon and wrapping it around a pencil. The device 50 is loaded onto shaft 82 by advancing the shaft 82 as cut device 50 is circumferentially wrapped around shaft 82 so that there is no overlap of the device 50 on itself, thereby following a helical pattern. An optional cover 85 is used for the catheter 80 to keep the wrapped and loaded device 50 compressed in its compressed, closed, pre-deployed or collapsed state.

One geometry, merely used as an example, is depicted in Figs. 5A, 5B and 5C, where the initial shape of device 50 appears to be cut out of a ribbon (Fig. 5A), but when expanded, one side/edge expands more than the other generating a circular path (Figs. 5B and 5C). When the circular path is given an axial component, the helical filter shape (helix 55) of filter device 50 is generated.

Accordingly, as shown in Fig. 6, the filter device 50 according to the present invention provides for an extremely compact delivery method thereby providing flexibility in the delivery method. The helical shape (helix 55, i.e. single helix or double helix design) inherently conforms to the shaft 82 of the catheter 80 and is able to achieve a tight bend radius as shown. Thus, the filter device 50 is self-centering and can easily adapt and function in a tightly constricted and bent environment.

Furthermore, variations for the filter device 50 are also contemplated herein according to the present invention. For example, as mentioned above, the helical turns 65 of the filter device 50 can have a variable pitch. Additionally, one end of the filter device 50 can coil in on itself, thereby providing an absolute type filter and eliminate any perception that a clot 20 may travel passed the filter 50.

Moreover, the filter device 50 is optionally coated with a drug such as a cytotoxic drug or cytostatic drug in order to make the filter device 50 a drug eluting device for treatment of disease that responds to cytotoxic drugs (for example paclitaxel) or cytostatic drugs (for example one of the rapamycins) respectively. As used herein, the term "drug" or "drugs" are used interchangeably herein and define an agent, drug, compound, composition of matter or mixture thereof which provides some therapeutic, often beneficial, effect such as being cytotoxic or cytostatic as two examples.

This includes pesticides, herbicides, germicides, biocides, algicides, rodenticides, fungicides, insecticides, antioxidants, plant growth promoters, plant growth inhibitors, preservatives, antipreservatives, disinfectants, sterilization agents, catalysts, chemical reactants, fermentation agents, foods, food supplements, nutrients, cosmetics, drugs, vitamins, sex sterilants, fertility inhibitors, fertility promoters, microorganism attenuators and other agents that benefit the environment of use. As used herein, the terms further include any physiologically or pharmacologically active substance that produces a localized or systemic effect or effects in animals, including warm blooded mammals, humans and primates; avians; domestic household or farm animals such as cats, dogs, sheep, goats, cattle, horses and pigs; laboratory animals such as mice, rats and guinea pigs; fish; reptiles; zoo and wild animals; and the like. The active drug that can be delivered includes inorganic and organic compounds, including, without limitation, drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system. Suitable agents may be selected from, for example, proteins, enzymes, hormones, polynucleotides, nucleoproteins, polysaccharides, glycoproteins, lipoproteins, polypeptides, steroids, hypnotics and sedatives, psychic energizers, tranquilizers, anticonvulsants, muscle relaxants, antiparkinson agents, analgesics, anti-inflammatories, local anesthetics, muscle contractants, blood pressure medications and cholesterol lowering agents including statins, antimicrobials, antimalarials, hormonal agents including contraceptives, sympathomimetics, polypeptides and proteins capable of eliciting physiological effects, diuretics, lipid regulating agents, antiandrogenic agents, antiparasitics, neoplastics, antineoplastics, hypoglycemics, nutritional agents and supplements, growth supplements, fats, ophthalmics, antienteritis agents, electrolytes and diagnostic agents.

Examples of the therapeutic agents or drugs useful in this invention include prochlorperazine edisylate, ferrous sulfate, aminocaproic acid, mecaxylamine hydrochloride, procainamide hydrochloride, amphetamine sulfate, methamphetamine hydrochloride, benzphetamine hydrochloride, isoproteronol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperazine maleate, anisindione, diphenadione, erythrityl tetranitrate, digoxin, isoflurophate, acetazolamide, methazolamide, bendroflumethiazide, chlorpropamide, tolazamide, chlormadinone acetate, phenaglycodol, allopurinol, aluminum aspirin, methotrexate, acetyl sulfisoxazole, hydrocortisone, hydrocorticosterone acetate, cortisone acetate, dexamethasone and its derivatives such as betamethasone, triamcinolone, methyltestosterone, 17-.beta.-estradiol, ethinyl estradiol, ethinyl estradiol 3-methyl ether, prednisolone, 17-.beta.-hydroxyprogesterone acetate, 19-nor-progesterone, norgestrel, norethindrone, norethisterone, norethiederone, progesterone, norgesterone, norethynodrel, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, theophylline, calcium gluconate, ketoprofen, ibuprofen, atorvastatin, simvastatin, pravastatin, fluvastatin, lovastatin, cephalexin, erythromycin, haloperidol, zomepirac, ferrous lactate, vincamine, phenoxybenzamine, diltiazem, milrinone, captropril, mandol, quanbenz, hydrochlorothiazide, ranitidine, flurbiprofen, fenbufen, fluprofen, tolmetin, alclofenac, mefenamic, flufenamic, difuninal, nimodipine, nitrendipine, nisoldipine, nicardipine, felodipine, lidoflazine, tiapamil, gallopamil, amlodipine, mioflazine, lisinopril, enalapril, captopril, ramipril, enalaprilat, famotidine, nizatidine, sucralfate, etintidine, tetratolol, minoxidil, chlordiazepoxide, diazepam, amitriptylin, and imipramine. Further examples are proteins and peptides which include, but are not limited to, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatropin, oxytocin, vasopressin, prolactin, somatostatin, lypressin, pancreozymin, luteinizing hormone, LHRH, interferons, interleukins, growth hormones such as human growth hormone, bovine growth hormone and porcine growth hormone, fertility inhibitors such as the prostaglandins, fertility promoters, growth factors, and human pancreas hormone releasing factor.

Moreover, drugs or pharmaceutical agents useful for the filter device 50 include: antiproliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP)II_{b}IIIₐ inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); antiinflammatory: such as adrenocortical steroids (cortisol, cortisone, fludrocortisonc, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetominophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF) platelet derived growth factor (PDGF), erythropoetin,; angiotensin receptor blocker; nitric oxide donors; anti-sense oligionucleotides and combinations thereof; cell cycle inhibitors, mTOR inhibitors, growth factor signal transduction kinase inhibitors, chemical compound, biological molecule, nucleic acids such as DNA and RNA, amino acids, peptide, protein or combinations thereof.

It is to be understood that the use of the term "drug" or drugs" includes all derivatives, analogs and salts thereof and in no way excludes the use of two or more such drugs.

The one or more drugs are coated on the filter device 50 itself or any desired portion of the device 50, for example, the outer circumferential edge of the helical turns 65. Moreover, the drug can be used with a polymer coating or the drug can be incorporated into the mesh material 52 of the device 50 itself when the mesh material 52 itself is made of a polymer material as mentioned above.

As shown in Figs. 2B, 3B and 4B, the filter device 50 alternatively has anchoring mechanisms 68 such as sharp edges or barbs along the outside periphery of the helix 55, i.e. the turns 65, in order to facilitate securing or anchoring into the vascular wall 12. Additionally, it is also contemplated that the device 50 according to the present invention have any other types of attachment mechanisms suited to the intended environment.

As mentioned above, the deployment mechanism for the filter device 50 may be due to the material 52 itself (when the material 52 is shape-memory material) and will be in the form of a helically wrapped tube (Fig. 6) or a compressed disc (not shown). The delivery device may be a structure solely made up of the compressed filter device 50 itself or alternatively the filter device 50 may be inserted in a delivery mechanism (e.g. a delivery tube or catheter 80 wherein the filter device is loaded in a compressed state between the shaft 82 and cover 85 of the catheter 80).

The mesh material 52 may be of any form, i.e. from a self-expanding material such as nitinol to a stainless steel material requiring a delivery mechanism to form it into its final shape, or it may be a polymer or blend of polymers, to name a few examples. The filter device is also made to be retractable (if desired). For instance, due to the nature of the helix design, by applying a twisting action reverse (reverse torque) to that which expanded the filter device when originally deployed in the vessel 10, the filter device 50 can be collapsed and retracted and withdrawn from the vessel 10 and the patient's body. The material 52 can also be of the type that requires a delivery mechanism to form filter device 50 into its final helical shape.

Inasmuch as the foregoing specification comprises preferred embodiments of the invention, it is understood that variations and modifications may be made herein, in accordance with the inventive principles disclosed, without departing from the scope of the invention.

While preferred embodiments of the present invention have been shown and described herein, it will be obvious to those skilled in the art that such embodiments are provided by way of example only. Numerous variations, changes and substitutions will now occur to those skilled in the art without departing from the invention. Accordingly, it is intended that the invention be limited only by the spirit and scope of the appended claims.

## Claims

1. A device (50) for capturing an embolus (20) within a vessel (10) of a patient's body, the device comprising:
at least one helicoid (55) made of a mesh material (52), the at least one helicoid having a plurality of turns (65) helicoidally arranged around a longitudinal axis of the device (50), the mesh material (52) having a plurality of pores (53) therein, the pores having a size ≥120 µm,
**characterised in that** the helicoid is defined by a helical line proximate to the longitudinal axis of the device and a helical line distal to the longitudinal axis of the device, and wherein in an expanded state the helical line proximate to the longitudinal axis of the device is roughly aligned with the longitudinal axis of the device.

2. The device according to Claim 1, wherein the at least one helicoid (55) comprises a plurality of panels (60).

3. The device according to Claim 2, wherein the panels (60) are movable from a collapsed state to an expanded state when placed within the vessel (10).

4. The device according to Claim 3, wherein the mesh panels (60) form the plurality of turns (65).

5. The device according to Claim 3 or Claim 4, wherein the mesh material (52) comprises a self-expanding material.

6. The device according to Claim 5, wherein the self-expanding material (52) comprises a shape memory material, which preferably comprises a metal alloy, such as nickel titanium.

7. The device according to Claim 3 or Claim 4, wherein the mesh material (52) comprises stainless steel.

8. The device according to Claim 5, wherein the self-expanding material (52) comprises a polymer.

9. The device according to Claim 8, wherein the polymer is biodegradable and preferably bioabsorbable.

10. The device according to Claim 8 or Claim 9, further comprising a drug.

11. The device according to Claim 10, wherein the drug is cytostatic, such as a rapamycin.

12. The device according to Claim 10, wherein the drug is cytotoxic, such as paclitaxel.

13. The device according to any preceding Claim, further comprising at least one anchoring mechanism (68), such as at least one barb, on the at least one helicoid (55).

14. The device according to any preceding Claim, wherein the at least one helicoid (55) comprises a double helicoid.

15. The device according to any preceding Claim, further comprising a spine (57).

16. The device according to any preceding Claim, wherein the pores (53) vary in size from one end of the at least one helicoid (55) to an opposite end of the at least one helicoid.

17. The device according to Claim 16, wherein the pores (53) vary in size from a larger size at the one end of the at least one helicoid (55) to a smaller size at the opposite end of the at least one helicoid.

18. The device according to any preceding Claim, wherein the pitch of the at least one helicoid (55)varies in size from one end of the at least one helicoid to an opposite end of the at least one helicoid.

19. The device according to Claim 18, wherein the pitch of the at least one helicoid (55) varies in size from a larger size at the one end of the at least one helicoid to a smaller size at the opposite end of the at least one helicoid.

## Patentansprüche

1. Vorrichtung (50) zum Ergreifen eines Embolus (20) in einem Gefäß (10) des Körpers eines Patienten, wobei die Vorrichtung Folgendes umfasst:
zumindest ein Helikoid (55) aus einem Netzmaterial (52), wobei das zumindest eine Helikoid eine Vielzahl von Windungen (65) aufweist, die schraubenlinienförmig um eine Längsachse der Vorrichtung (50) angeordnet sind, wobei das Netzmaterial (52) eine Vielzahl von Poren (53) darin aufweist, wobei die Poren eine Größe ≥120 µm aufweisen,
**dadurch gekennzeichnet, dass** das Helikoid durch eine Schraubenlinie neben der Längsachse der Vorrichtung und eine Schraubenlinie distal zur Längsachse der Vorrichtung definiert wird, und worin in einem expandierten Zustand die Schraubenlinie neben der Längsachse der Vorrichtung ungefähr mit der Längsachse der Vorrichtung ausgerichtet ist.

2. Vorrichtung nach Anspruch 1, worin das zumindest eine Helikoid (55) eine Vielzahl von Tafeln (60) umfasst.

3. Vorrichtung nach Anspruch 2, worin die Tafeln (60) aus einem zusammengeklappten Zustand in einen expandierten Zustand bewegbar sind, wenn sie in dem Gefäß (10) platziert sind.

4. Vorrichtung nach Anspruch 3, worin die Netztafeln (60) eine Vielzahl von Windungen (65) bilden.

5. Vorrichtung nach Anspruch 3 oder Anspruch 4, worin das Netzmaterial (52) ein selbst-expandierendes Material umfasst.

6. Vorrichtung nach Anspruch 5, worin das selbstexpandierende Material (52) ein Formgedächtnismaterial umfasst, das vorzugsweise eine Metalllegierung, wie etwa Nickel-Titan, umfasst.

7. Vorrichtung nach Anspruch 3 oder Anspruch 4, worin das Netzmaterial (52) Edelstahl umfasst.

8. Vorrichtung nach Anspruch 5, worin das selbstexpandierende Material (52) ein Polymer umfasst.

9. Vorrichtung nach Anspruch 8, worin das Polymer biologisch abbaubar und vorzugsweise bioresorbierbar ist.

10. Vorrichtung nach Anspruch 8 oder Anspruch 9, ferner ein Arzneimittel umfassend.

11. Vorrichtung nach Anspruch 10, worin das Arzneimittel zytostatisch ist, beispielsweise ein Rapamycin.

12. Vorrichtung nach Anspruch 10, worin das Arzneimittel zytotoxisch ist, beispielsweise Placlitaxel.

13. Vorrichtung nach einem der vorhergehenden Ansprüche, ferner zumindest einen Verankerungsmechanismus (68), wie etwa zumindest einen Widerhaken, auf dem zumindest einen Helikoid (55) umfassend.

14. Vorrichtung nach einem der vorhergehenden Ansprüche, worin das zumindest eine Helikoid (55) ein Doppel-Helikoid umfasst.

15. Vorrichtung nach einem der vorhergehenden Ansprüche, ferner ein Rückgrat (57) umfassend.

16. Vorrichtung nach einem der vorhergehenden Ansprüche, worin die Größe der Poren (53) von einem Ende des zumindest einen Helikoids (55) zu einem gegenüberliegenden Ende des zumindest einen Helikoids variiert.

17. Vorrichtung nach Anspruch 16, worin die Größe der Poren (53) von einer größeren Größe an dem einen Ende des zumindest einen Helikoids (55) zu einer kleineren Größe an dem gegenüberliegenden Ende des zumindest einen Helikoids variiert.

18. Vorrichtung nach einem der vorhergehenden Ansprüche, worin die Größe der Steigung des zumindest einen Helikoids (55) von einem Ende des zumindest einen Helikoids zu einem gegenüberliegenden Ende des zumindest einen Helikoids variiert.

19. Vorrichtung nach Anspruch 18, worin die Größe der Steigung des zumindest einen Helikoids (55) von einer größeren Größe an dem einen Ende des zumindest einen Helikoids zu einer kleineren Größe an dem gegenüberliegenden Ende des zumindest einen Helikoids variiert.

## Revendications

1. Dispositif (50) pour capturer un embole (20) dans un vaisseau (10) du corps d'un patient, le dispositif comprenant :
au moins un hélicoïde (55) fait d'une matière à mailles (52), ledit hélicoïde comportant une pluralité de tours (65) disposés en hélice autour d'un axe longitudinal du dispositif (50), la matière à mailles (52) comportant en elle une pluralité de pores (53), les pores ayant une dimension ≥ 120 µm, **caractérisé en ce que** l'hélicoïde est défini par une ligne hélicoïdale proximale à l'axe longitudinal du dispositif et une ligne hélicoïdale distale à l'axe longitudinal du dispositif et dans lequel, dans un état dilaté, la ligne hélicoïdale proximale à l'axe longitudinal du dispositif est à peu près alignée avec l'axe longitudinal du dispositif.

2. Dispositif selon la revendication 1, dans lequel ledit hélicoïde (55) comprend une pluralité de panneaux (60).

3. Dispositif selon la revendication 2, dans lequel les panneaux (60) sont mobiles depuis un état affaissé jusqu'à un état dilaté quand ils sont placés à l'intérieur du vaisseau (10).

4. Dispositif selon la revendication 3, dans lequel les panneaux (60) à mailles constituent la pluralité de tours (65).

5. Dispositif selon la revendication 3 ou 4, dans lequel la matière à mailles (52) comprend une matière auto-extensible.

6. Dispositif selon la revendication 5, dans lequel la matière auto-extensible (52) comprend une matière à mémoire de forme, qui comprend de préférence un alliage métallique, tel que nickel-titane.

7. Dispositif selon la revendication 3 ou 4, dans lequel la matière à mailles (52) comprend de l'acier inoxydable.

8. Dispositif selon la revendication 5, dans lequel la matière auto-extensible (52) comprend un polymère.

9. Dispositif selon la revendication 8, dans lequel le polymère est biodégradable et de préférence bioabsorbable.

10. Dispositif selon la revendication 8 ou 9, comprenant en outre un médicament.

11. Dispositif selon la revendication 10, dans lequel le médicament est cytostatique, par exemple de la rapamycine.

12. Dispositif selon la revendication 10, dans lequel le médicament est cytostatique, par exemple du paclitaxel.

13. Dispositif selon l'une quelconque des revendications précédentes, comprenant en outre au moins un mécanisme d'ancrage (68), par exemple au moins une barbe, sur ledit hélicoïde (55).

14. Dispositif selon l'une quelconque des revendications précédentes, dans lequel ledit hélicoïde (55) comprend un double hélicoïde.

15. Dispositif selon l'une quelconque des revendications précédentes, comprenant en outre une épine dorsale (57).

16. Dispositif selon l'une quelconque des revendications précédentes, dans lequel les pores (53) varient en dimension d'une extrémité dudit hélicoïde (55) à l'extrémité opposée dudit hélicoïde.

17. Dispositif selon la revendication 16, dans lequel les pores (53) varient en dimension d'une dimension plus grande à la première extrémité dudit hélicoïde (55) à une dimension plus faible à l'extrémité opposée dudit hélicoïde.

18. Dispositif selon l'une quelconque des revendications précédentes, dans lequel le pas dudit hélicoïde (55) varie en dimension d'une extrémité dudit hélicoïde à l'extrémité opposée dudit hélicoïde.

19. Dispositif selon la revendication 18, dans lequel le pas dudit hélicoïde (55) varie en dimension d'une dimension plus grande à la première extrémité dudit hélicoïde à une dimension plus faible à l'extrémité opposée dudit hélicoïde.
